# EUROPÄISCHE PATENTSCHRIFT

(11) **EP 0 945 405 B1**
(45) Veröffentlichungstag und Bekanntmachung des Hinweises auf die Patenterteilung: **18.12.2002**
(21) Anmeldenummer: 99103500.7
(22) Anmeldetag: 24.02.1999
(51) Int. Cl.: C02F 1/72, C02F 1/50, C02F 5/10

(54) **Härtestabilisierende Percarbonsäurelösungen, Verfahren zu deren Herstellung und deren Verwendung**
Solutions of peracids for stabilizing hardness, method of preparation, and their use
Solutions de peracides pour stabiliser la dureté,procédé de préparation et leur utilisation

(30) Priorität: 23.03.1998 DE 19812588
(43) Veröffentlichungstag der Anmeldung: 29.09.1999
(73) Patentinhaber: Degussa AG, 40474 Düsseldorf (DE)
(72) Erfinder: Reinold, Andreas, 63584 Gründau (DE); Walzer, Egon, Dr., 63477 Maintal (DE)

(56) Entgegenhaltungen:
- US-A- 4 997 571

## Beschreibung

Die Erfindung betrifft härtestabilisierende Percarbonsäurelösungen, enthaltend eine oder mehrere Percarbonsäuren aus der Reihe Perameisensäure, Peressigsäure und Perpropionsäure, die den Percarbonsäuren zugrundeliegenden Carbonsäuren, Wasserstoffperoxid, Wasser und einen Härtestabilisator. Die Erfindung richtet sich ferner auf ein Verfahren zur Herstellung der härtestabilisierenden Percarbonsäurelösungen sowie auf deren Verwendung.

Percarbonsäuren (= Peroxycarbonsäuren), wie insbesondere Peressigsäure und Perameisensäure, sind hochwirksame Mikrobiozide, die in unterschiedlichsten Wassersystemen zur Bekämpfung von Mikroorganismen Anwendung finden - beispielhaft wird auf die WO 97/08100 und die EP 0 688 302 verwiesen. Besonders effizient werden die genannten Percarbonsäuren in Form einer sogenannten wäßrigen Gleichgewichtspercarbonsäurelösung eingesetzt, welche im wesentlichen aus einer Percarbonsäure, der zugrundeliegenden Carbonsäure, Wasserstoffperoxid und Wasser bestehen und zusätzlich einen Mineralsäurekatalysator und übliche Aktivsauerstoffstabilisatoren in wirksamer Menge enthalten können.

Um anwendungsspezifischen Anforderungen gerecht zu werden, werden den genannten Percarbonsäurelösungen Zusatzstoffe zugefügt, wie Netzmittel, Emulgatoren, Korrosionsinhibitoren und Puffer. Die mikrobioziden Zusammensetzungen gemäß WO 93/10088 bzw. WO 94/14321 basieren auf einer Gleichgewichts-Percarbonsäure und nichtionischen Tensiden. Eine ähnliche Zusammensetzung, jedoch mit einem anionischen Tensid aus der Reihe der Alkylbenzolsulfonate, Alkylsulfate und Alkansulfonate anstelle des nichtionischen Tensids, ist aus der FR 2 321 301 und DE 26 16 049 bekannt, wobei die Zusammensetzung des letztgenannten Dokuments zusätzlich eine Phosphonsäure enthält. Eine stabilisierte wäßrige Peressigsäurelösung mit erhöhter antimikrobieller Wirkung und verminderter Korrosionswirkung enthält gemäß DD 96833 Pufferungsmittel, Netzmittel oder Emulgatoren, wie Alkalisalze von sulfonierten Fettsäuren, Fettalkoholsulfonaten und Alkanund Alkylarylsulfonaten, und organische Komplexbildner, wie Dipicolinsäure und Ethylendiamintetraessigsäure. Schließlich sind auch verdickte Percarbonsäurelösungen bekannt, etwa solche gemäß EP 0 421 974 B1, welche ein vernetztes Acrylsäurepolymer und zusätzlich ein Sequestriermittel aus der Reihe der Phosphonsäuren und stickstoffhaltigen Carbonsäuren enthalten.

In Wasserkreisläufen besteht vielfach die Aufgabe, gleichzeitig Störungen durch den Befall von Mikroorganismen und die Ausbildung fester Beläge aus Härtebildnern zu vermeiden oder zu mindern. Bisher wurden für diesen Zweck ein Härtestabilisator, insbesondere ein solcher aus der Reihe der Phosphonocarbonsäuren, und eine Gleichgewichtsperessigsäure getrennt dem Kreislaufsystem zudosiert. Diese Zudosierung war aufwendig. Eine Lösung aus einer Gleichgewichtsperessigsäure und einer Phosphonocarbonsäure zeigt zwar die geforderte mikrobiozide und härtestabilisierende Wirkung, die Lagerstabilität war aber unzureichend. Demgemäß bestand die Aufgabe der vorliegenden Erfindung darin, ein Mittel aufzuzeigen, das sowohl die mikrobiozide als auch die härtestabilisierende und belagverhindernde Wirkung in einem Mittel vereinigt. Gleichzeitig sollte das Mittel eine hohe Lagerstabilität aufweisen.

Gefunden wurde eine härtestabilisierende Percarbonsäurelösung, enthaltend eine oder mehrere Percarbonsäuren aus der Reihe der Perameisensäure, Peressigsäure und Perpropionsäure, die der/den Persäure/n zugrundeliegende/n Carbonsäuren, Wasserstoffperoxid, Wasser und einen Härtestabilisator aus der Reihe von (i) durch oxidative Polymerisation von Acrolein oder von Acrolein und Acrylsäure hergestellten Polymeren, (ii) Polyacrylsäure und (iii) Copolymeren aus Acrylsäure und einer anderen ungesättigten Carbonsäure, insbesondere Maleinsäure, und (iv) Polymaleinsäure, wobei der Härtestabilisator unvernetzt ist, das mittlere Molekulargewicht M_{w} des Härtestabilisators im Bereich von 500 bis 25000, insbesondere 1000 bis 15000, liegt und Carboxylgruppen des Härtestabilisators teilweise in Percarboxylgruppen überführt worden sein können.

Die Unteransprüche richten sich auf bevorzugte Ausführungsformen.

Bevorzugte härtestabilisierende Percarbonsäurelösungen enthalten als Percarbonsäure Peressigsäure oder eine Kombination aus Peressigsäure und Perameisensäure in einer Gesamtmenge von 0,1 bis 15 Gew.-%, insbesondere 0,5 bis 6 Gew.-%. Da die Härtestabilisatoren auch Carboxylgruppen enthalten, können diese durch anwesendes Wasserstoffperoxid teilweise in Percarbonsäuregruppen überführt worden sein. Zweckmäßigerweise ist die Lösung wäßrig und weist einen Wassergehalt über 40 Gew.-% auf. Die Percarbonsäure, die zugrundeliegende Carbonsäure, Wasserstoffperoxid und Wasser liegen vorzugsweise im Gleichgewichtszustand oder in der Nähe desselben vor. Die erfindungsgemäße Lösung enthält im allgemeinen 0,01 bis 10 Gew.-% Härtestabilisator (berechnet 100 %ig), vorzugsweise 0,5 bis 5 Gew.-%. Zusätzlich können auch weitere Zusatzstoffe zur Einstellung bestimmter auf die Anwendung gerichteter Zuatzstoffe enthalten sein. Unter den Zusatzstoffen sind beispielhaft zu nennen: Tenside, insbesondere anionische und nichtionische Tenside mit benetzender, emulgierender und/oder dispergierender Wirkung, Korrosionsschutzmittel, Puffer, übliche Aktivsauerstoffstabilisatoren, wie Phosphonsäurederivate, Pyridincarbonsäuren, Amino- und Hydroxycarbonsäuren, Zinnverbindungen und Radikalfänger, und Mineralsäuren für die Gleichgewichtseinstellung, wie Schwefelsäure, Phosphorsäure, Pyro- und Polyphosphorsäure.

Die Verwendung von Aminocarbonsäuren, wie Ethylendiaminotetraessigsäure (EDTA) und Aminophosphonsäuren als Komplexbildner zum Abfangen von in Percarbonsäurelösungen enthaltenden Schwermetallen, die die Lagerstabilität von Aktivsauerstoffverbindungen mindern, ist an sich bekannt, jedoch sind derartige Stoffe einerseits aus ökotoxikologischer Sicht in größerer Menge unerwünscht und/oder ihre härtestabilisierende Wirkung ist nicht ausreichend. Zudem eignen sich derartige Substanzen unzureichend als Belagverhinderer; gerade in wäßrigen Kreislaufsystemen ist aber eine Belagverhinderung erforderlich. Bisher wurden in Wasserkreisläufen zur Bekämpfung von Mikroorganismen und niederen pflanzlichen und tierischen Organismen und zur Belagverhinderung eine Phosphonocarbonsäure, insbesondere 2-Phosphonobutan-1,2,4-tricarbonsäure (PBTC), getrennt eingesetzt. Die Zugabe von PBTC zu einer Gleichgewichtsperessigsäure führt aber, wie Vergleichsversuche zeigten, zu nicht ausreichend lagerstabilen Lösungen, die damit nicht marktfähig sind. Die erfindungsgemäßen Lösungen zeichnen sich demgegenüber durch eine hohe Lagerstabilität aus.

Es wurde ferner gefunden, daß anionische Tenside enthaltende Gleichgewichts-Peressigsäurelösungen beim Verdünnen mit Leitungswasser durch die Ausfällung von Kalkseifen stark trüb werden. Dieses Problem läßt sich weitgehend mindern, wenn die Lösung außer dem anionischen Tensid zusätzlich einen erfindungsgemäßen Härtestabilisator in wirksamer Menge enthält. Derartige Percarbonsäurelösungen enthalten vorzugsweise 0,5 bis 6 Gew.-% Peressigsäure oder eine Kombination aus Peressigsäure und Perameisensäure, 1 bis 10 Gew.-%, insbesondere 1 bis 7,5 Gew.-%, erfindungsgemäße Härtestabilisatoren (berechnet 100 %ig), insbesondere solche gemäß (i), und 0 bis 5 Gew.-% Tenside, insbesondere anionische Tenside auf Sulfonsäure- und Sulfatesterbasis.

Die erfindungsgemäß in den Percarbonsäurelösungen enthaltenen Härtestabilisatoren wirken sowohl als Härtestabilisator als auch als Belagverhinderer. Produkte mit M_{w} kleiner 500 und größer 25000 sind weniger geeignet, da entweder die Wirkung und/oder die Löslichkeit zu gering sind. Bevorzugt werden Produkte mit einem Molekulargewicht M_{w} im Bereich von 500 bis 15000, insbesondere 1000 bis 10000. Bei der Stoffklasse gemäß (i) handelt es sich um carboxylgruppenhaltige Polymere, hergestellt durch Polymerisation von Acrolein, vorzugsweise aber von Acrolein und Acrylsäure in Gegenwart von Wasserstoffperoxid. Derartige Produkte sind unter der Bezeichnung POC (Firma Degussa AG) im Handel erhältlich, zum Beispiel POC HS2020 mit einem Molekulargewicht M_{w} um 9000. Härtestabilisatoren gemäß (ii) sind Polyacrylsäuren und gegebenenfalls modifizierte Polyacrylsäuren, vorzugsweise solche mit einem mittleren Molekulargewicht M_{w} von etwa 1000 bis 10000. Auch Copolymere auf der Basis von Acrylsäure mit einer anderen olefinischen Carbonsäure, wobei bevorzugt mindestens eine Carboxylgruppe an einem olefinischen C-Atom gebunden ist, wie Maleinsäure und Polymaleinsäure, sind geeignet, wenn M_{w} im bevorzugten Bereich von 500 bis 15000 liegt. Härtestabilisatoren gemäß (i), (ii), (iii) und (iv) können herstellungsbedingt eine geringe Menge an niederen Carbonsäuren und/oder Spuren Acrylsäure enthalten. Die als Härtestabilisator zu verwendenden Polymeren sollen im wesentlichen unvernetzt sein und zu keiner nennenswerten Verdickung der Percarbonsäurelösung führen. Üblicherweise liegt die Viskosität der erfindungsgemäßen Lösungen unter 20 mPa^{·}s.

Außer den auf den Härtestabilisator zurückzuführenden härtestabilisierenden, belagverhindernden und trübungsminderenden Wirkungen, zeichnen sich die zu verwendenden Härtestabilisatoren auch dadurch aus, daß sie die Aktivsauerstoffstabilität der Percarbonsäurelösung erhöhen.

Die Herstellung der erfindungsgemäßen Percarbonsäurelösungen erfolgt am einfachsten durch Zugabe des Härtestabilisators zu einer zuvor in üblicher Weise hergestellten Percarbonsäurelösung, insbesondere einer Gleichgewichtspercarbonsäurelösung. Durch Stehenlassen stellt sich ein neues Gleichgewicht ein. Es ist auch möglich, den Härtestabilisator vor der Herstellung der Percarbonsäurelösung einem der Reaktionspartner, also der Carbonsäure, dem Wasserstoffperoxid oder dem Wasser, oder einem Gemisch hiervon zuzugeben und aus den entsprechenden Lösungen die Percarbonsäurelösung herzustellen.

Die erfindungsgemäßen Percarbonsäurelösungen lassen sich insbesondere zum Vorbeugen gegen und zum Bekämpfen von schädlichen Mikroorganismen, wie Pilze, Viren, Bakterien, Hefen und Algen, sowie Würmern, Muscheln und Insekten, in wäßrigen Systemen, insbesondere Wasserkreisläufen, und zur Oberflächendesinfektion verwenden.

### Beispiele B1 bis B3 und Vergleichsbeispiele VB1 bis VB3

Ausgehend von einer Gleichgewichts-Peressigsäurelösung mit einem Gehalt von 1,91 Gew.-% Peressigsäure (PES), 45,08 Gew.-% H₂O₂ und einem Aktivsauerstoffgehalt (AO) von 21,62 Gew.-% wurden Mischungen mit 5, 15 und 25 Gew.-% Bayhibit AM, einer 50 gew.-%igen Handelsware von 2-Phosphono-butan-1,2,4-Tricarbonsäure der Firma Bayer AG, hergestellt (VB1 bis VB3). In gleicher Weise wurden Mischungen der Peressigsäurelösung mit 5, 15 und 25 Gew.-% POC 2020, einer 50 gew.-%igen wäßrigen Lösung eines Härtestabilisators gemäß (i) der Firma Degussa AG, hergestellt (B1 bis B3). Die Gleichgewichtseinstellung und Stabilität wurden über die Bestimmung des PES-, H₂O₂- und AO-Gehalts bestimmt; die Bestimmungen erfolgten in bekannter Weise unter Verwendung von Cer(IV)-sulfat und Natriumthiosulfat.

**Tabelle 1**

| Mischungen mit POC 2020 als Härtestabilisator | | | | | |
|---|---|---|---|---|---|
| Nr. | Menge POC 2020 (Gew.-%) | Lagerzeit T = Tage W = Wochen | PES *) (Gew.-%) | H₂O₂ (Gew.-%) | AO (Gew.-%) |
| B1 | 5 | Start | 2,24 | 44.77 | 21,52 |
| | | 1 T | 2,38 | 44,70 | 21,53 |
| | | 4 T | 2,49 | 44,17 | 21,28 |
| | | 1 W | 2,78 | 44,02 | 21,27 |
| | | 2 W | 2,80 | 44,24 | 21,38 |
| | | 65 W | 2,81 | 42,35 | 20,48 |
| | | 93 W | 2,85 | 41,61 | 20,15 |
| B2 | 15 | Start | 2,69 | 39,49 | 19,44 |
| | | 1 T | 2,85 | 39,47 | 19,16 |
| | | 4 T | 3,18 | 39,11 | 19,06 |
| | | 1 W | 3,39 | 38,79 | 18,96 |
| | | 2 W | 3,99 | 38,76 | 19,06 |
| | | 65 W | 3,77 | 33,70 | 16,62 |
| | | 93 W | 2,79 | 30,27 | 14,89 |
| B3 | 25 | Start | 2,87 | 35,00 | 17,05 |
| | | 1 T | 3,37 | 34,42 | 16,89 |
| | | 4 T | 3,90 | 34,04 | 16,82 |
| | | 1 W | 4,15 | 33,78 | 16,76 |
| | | 2 W | 4,48 | 33,46 | 16,67 |
| | | 65 W | 3,91 | 26,25 | 13,15 |
| | | 93 W | 2,94 | 22,63 | 11,25 |

| | | | | | |
|---|---|---|---|---|---|
| *) Summe aller Percarbonsäuren, berechnet als Peressigsäure | | | | | |

**Tabelle 2**

| Mischungen mit Bayhibit AM (PBTC) als Härtestabilisator | | | | | |
|---|---|---|---|---|---|
| Nr. | Menge PBTC (Gew.-%) | Lagerzeit T = Tage W = Wochen | PES *) (Gew.-%) | H₂O₂ (Gew.-%) | AO (Gew.-%) |
| VB1 | 5 | Start | 2,25 | 44,64 | 21,45 |
| | | 1 T | 2,29 | 44,52 | 21,41 |
| | | 4 T | 2,38 | 44,19 | 21,28 |
| | | 1 W | 2,60 | 44,38 | 21,43 |
| | | 2 W | 2,69 | 44,34 | 21,42 |
| | | 65 W | 2,29 | 41,39 | 19,92 |
| | | 93 W | 2,15 | 40,21 | 19,34 |
| VB2 | 15 | Start | 2,77 | 39,53 | 19,16 |
| | | 1 T | 2,98 | 39,47 | 19,18 |
| | | 4 T | 3,30 | 39,29 | 19,16 |
| | | 1 W | 3,41 | 38,86 | 18,82 |
| | | 2 W | 3,67 | 39,03 | 19,12 |
| | | 65 W | 2,41 | 30,41 | 14,79 |
| | | 93 W | 1,67 | 25,04 | 12,09 |
| VB3 | 25 | Start | 3,20 | 34,30 | 16,81 |
| | | 1 T | 3,54 | 34,42 | 16,93 |
| | | 4 T | 3,81 | 34,09 | 16,80 |
| | | 1 W | 4,26 | 33,84 | 16,74 |
| | | 2 W | 4,20 | 33,65 | 16,70 |
| | | 65 W | 1,23 | 14,95 | 7,28 |
| | | 93 W | 0,35 | 5,82 | 2,81 |

| | | | | | |
|---|---|---|---|---|---|
| *) Summe aller Percarbonsäuren, berechnet als Peressigsäure | | | | | |

In B1 bis B3 und VB1 bis VB3 findet innerhalb von etwa 2 Wochen nach Zugabe des Härtestabilisators die Gleichgewichtseinstellung statt. Danach steigt aber der Percarbonsäuregehalt noch etwas weiter an, offensichtlich aufgrund einer Reaktion von H₂O₂ mit Carboxylgruppen des Härtestabilisators. Die Zugabemenge an Härtestabilisator beeinflußt den Maximalwert an Percarbonsäuren. Der gesamte Percarbonsäuregehalt war in den Beispielen B1 bis B3 nach 2 Wochen etwas größer als in den Vergleichsbeispielen VB1 bis VB3; die Ursache wird darin gesehen, daß die Carboxylgruppen des eingesetzten POC 2020 und/oder der darin enthaltenen Nebenbestandteile teilweise in Peroxycarboxylgruppen überführt wurden.

Die erfindungsgemäßen Lösungen weisen eine höhere Aktivsauerstoffstabilität auf als die Lösungen der Vergleichsbeispiele. Die geringere Lagerstabilität der nicht-erfindungsgemäßen Lösungen zeigt sich besonders bei höheren Konzentrationen an Härtestabilisator.

### Beispiel B4 und Vergleichsbeispiel VB4

Untersucht wurde die Wirkung des Härtestabilisators /Belagverhinderers POC 2020 in einer ein anionischens Tensid enthaltenden Gleichgewichts-Peressigsäure beim Verdünnen der Lösung mit Wasser mit standardisierter Härte (21,28 °d). Eingesetzt wurde eine Gleichgewichts-Peressigsäure mit 5 Gew.-% PES (= PES 5 %), zugegeben wurden die in der Tabelle angegebenen Mengen an Tensid Hostapur SAS 60 (sekundäres Alkansulfonat-Na-Salz 60 %ig der Firma Hoechst AG) und POC 2020; die in der Tabelle angegebenen Mengen an SAS 60 und POC 2020 sind als 100 % Wirksubstanz angegeben und bezogen auf die Lösung. Verdünnt wurden die Lösungen auf eine PES-Konzentration von 0,5 Gew.-% beziehungsweise 1 Gew.-%. Die Zusammensetzungen der Lösungen und die Trübungswerte folgen aus der Tabelle 3.

**Tabelle 3**

| Nr. | Zusammensetzung | Trübung beim Verdünnen | |
|---|---|---|---|
| | | auf 0,5 % PES | auf 1 % PES |
| VB4 | PES 5 % + 3 % SAS 60 | stark | sehr stark |
| | | | |
| B4/1 | PES 5 % + 1 % SAS 60 + 1 % POC 2020 | schwach | mittel |
| | | | |
| B4/2 | PES 5 % + 3 % SAS 60 + 3 % POC 2020 | schwach / mittel | mittel |
| | | | |
| B4/3 | PES 5 % + 1 % SAS 60 + 3 % POC 2020 | sehr schwach | schwach |

Durch die Zugabe eines erfindungsgemäßen Härtestabilisators kann der Trübungsgrad einer ein anionisches Tensid enthaltenden Peressigsäurelösung beim Verdünnen mit hartem Wasser wesentlich reduziert werden.

### Beispiel B5 und Vergleichsbeispiel VB5

Bestimmt wurde die Wirkung von POC 2020 als Aktivsauerstoffstabilisator in einer Gleichgewichts-Peressigsäure (= B5) im Vergleich zu einer unstabilisierten und mit anderen Stabilisatoren stabilisierten PES-Lösung.

Hergestellt wurden wäßrige Peressigsäurelösungen aus 27,9 Gew.-% H₂O₂, 28,1 Gew.-% Essigsäure, 2 Gew.-% Polyphosphorsäure, den entsprechenden Stabilisatoren und Wasser (Ergänzung auf 100 %). Die Lösungen wurden 7 Tage gelagert, dann wurde zu je 320 g Lösung ein V4A-Edelstahlcoupon (Werkstoff 1.4571) zugegeben, entsprechend einer Belastung von 13 ml/cm². Nach 14 Tagen wurden wieder die Gehalte an PES und H₂O₂ sowie die Zersetzungsraten (140 g, 1 h Aufheizen auf 60 °C, 30 min O₂-Entwicklung messen) bestimmt. Die Ergebnisse folgen aus der Tabelle 4.

Die erfindungsgemäß zu verwendenden Härtestabilisatoren eignen sich auch als Aktivsauerstoffstabilisator.

**Tabelle 4**

| Nr. | Stabilisator *) | Zersetzungsrate (ml O₂) nach 7 Tagen Reifung | Gehalte (Gew.-%) und Zersetzungsrate (ml O₂) nach 14 Tagen Lagerung der gereiften Lösungen mit V4A-Belastung | | |
|---|---|---|---|---|---|
| | | | % PES | % H₂O₂ | ml O₂ |
| VB 5.1 | ohne | n.b. | 15,06 | 21,38 | 3,6 |
| | | | | | |
| VB 5.2 | mit 100 ppm DPS + 400 ppm HEDP | 0,1 | 15,44 | 21,91 | 1,3 |
| | | | | | |
| B 5.1 | mit 100 ppm POC 2020 | 0,6 | 15,31 | 21,26 | 3,0 |
| | | | | | |
| B 5.2 | mit 500 ppm POC 2020 | 0,1 | 15,25 | 21,24 | 1,9 |
| DPS = Dipicolinsäure HEDP = Hydroxyethandiphosphonsäure | | | | | |

| | | | | | |
|---|---|---|---|---|---|
| *) alle Konzentrationsangaben beziehen sich auf 100 % Wirksubstanz | | | | | |

### Beispiele B6 bis B11

Hergestellt wurden 5 gew.-%ige Gleichgewichts-Peressigsäuren, enthaltend unterschiedliche Härtestabilisatoren (Beispiele 6 bis 8) sowie die gleichen Härtestabilisatoren in Verbindung mit einem anionischen Tensid (Beispiele 9 bis 11) - siehe Tabelle 5. Die Herstellung erfolgte durch Mischen von 28,05 % H₂O₂ (berechnet 100 %), 9,07 % Essigsäure (berechnet 100 %), 0,97 % H₂SO₄ (= Katalysator), 0,05 % Dipicolinsäure (= Stabilisator), den in der Tabelle angegebenen Mengen Härtestabilisator und Tensid und Ergänzung mit Wasser auf 100 %. Die Gemische wurden in Glasflaschen gelagert und nach 5 und 42 Tagen analysiert - die Gehalte an PES, H₂O₂ und AO folgen aus der Tabelle 5.

Gemessen am Verlust an Aktivsauerstoff (AO) zeigten die Proben mit Sokolan CP 10S (BASF AG), ein Härtestabilisator gemäß (ii), die höchste Lagerstabilität. Die Carboxylgruppen des Härtestabilisators Belgard EV (Ciba Geigy AG), ein Härtestabilisator gemäß (iv), reagieren in erheblichem Umfang mit Wasserstoffperoxid zu Percarboxylgruppen: hieraus resultiert der hohe PES-Wert in Beispiel 8; überraschenderweise wird diese Persäurebildung durch anwesendes Tensid weitgehend verhindert - siehe Beispiel 11. (Die Angabe Gew.-% PES in der Tabelle umfaßt alle Percarbonsäuregruppen, berechnet wurden sie aber als Peressigsäure).

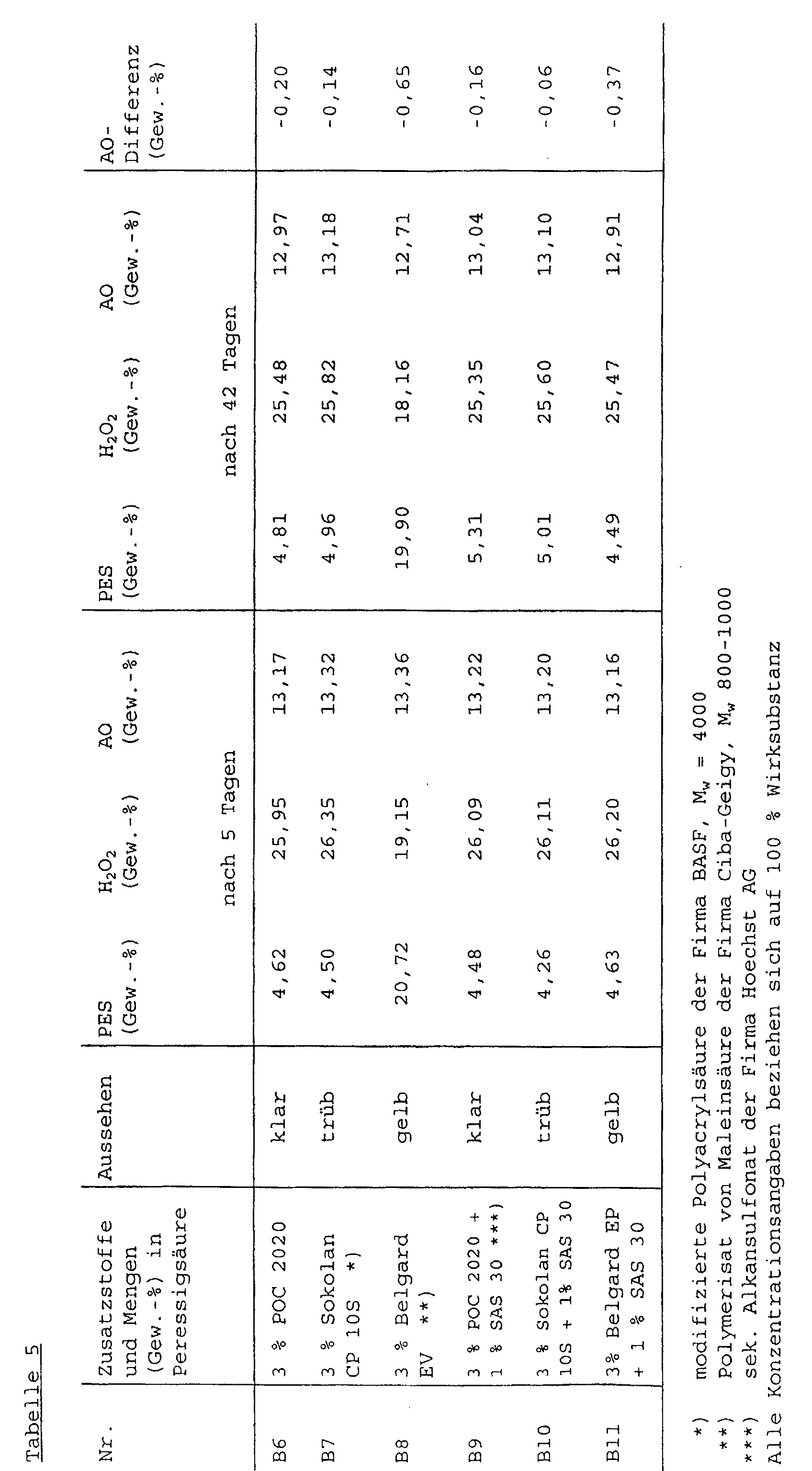

### Beispiele B12 bis B17

Bestimmung des Kalkbindevermögens (KBV) von 15 Gew.-%iger Gleichgewichts-Peressigsäure (G-PES 15), enthaltend verschiedene Härtestabilisatoren (Beispiele 12 bis 14) sowie Härtestabilisatoren in Verbindung mit einem anionischen Tensid (Beispiele 15 bis 17). Der Tabelle 6 sind die Zusammensetzungen sowie das Kalkbindevermögen in mg CaCO₃ pro g Härtestabilisator, bestimmt mittels Trübungstitration, zu entnehmen. Es zeigte sich, daß durch die Gegenwart des anionischen Tensids das KBV stark abnahm.

**Tabelle 6**

| Nr. | Zusatzstoffe in G-PES 15 | Kalkbindevermögen (mg CaCO₃/g) nach 88 Tagen Lagerung |
|---|---|---|
| B12 | 3 % POC 2020 | 2560 |
| | | |
| B13 | 3 % Sokolan CP 10S | 3170 |
| | | |
| B14 | 3 % Belgard EP | 1820 |
| | | |
| B15 | 3 % POC 2020 + 1 % SAS 30 | 830 |
| | | |
| B16 | 3 % Sokolan CP 10S + 1 % SAS 30 | 1650 |
| | | |
| B17 | 3 % Belgard EV + 1 % SAS 30 | 990 |
| *) modifizierte Polyacrylsaure der Firma BASF, M_{w} = 4000 | | |
| **) Polymerisat von Maleinsäure der Firma Ciba-Geigy, M_{w} 800-1000 | | |
| ***) sek. Alkansulfonat der Firma Hoechst AG | | |

Alle Konzentrationsangaben beziehen sich auf 100 % Wirksubstanz

## Patentansprüche

1. Härtestabilisierende Percarbonsäurelösung, enthaltend eine oder mehrere Percarbonsäuren aus der Reihe der Perameisensäure, Peressigsäure und Perpropionsäure, die der/den Persäure/n zugrundeliegende/n Carbonsäuren, Wasserstoffperoxid, Wasser und einen Härtestabilisator aus der Reihe von (i) durch oxidative Polymerisation von Acrolein oder von Acrolein und Acrylsäure hergestellten Polymeren, (ii) Polyacrylsäure, (iii) Copolymeren aus Acrylsäure und einer anderen ungesättigten Carbonsäure, insbesondere Maleinsäure, und (iv) Polymaleinsäure, wobei der Härtestabilisator unvernetzt ist, das mittlere Molekulargewicht M_{w} des Härtestabilisators im Bereich von 500 bis 25000, insbesondere 1000 bis 15000, liegt und Carboxylgruppen des Härtestabilisators teilweise in Percarboxylgruppen überführt worden sein können.

2. Härtestabilisierende Percarbonsäurelösung nach Anspruch 1,
**dadurch gekennzeichnet,**
**daß** sie 0,1 bis 10 Gew.-% Härtestabilisator enthält.

3. Härtestabilisierende Percarbonsäurelösung nach Anspruch 1 oder 2, bestehend aus: 0,1 bis 15 Gew.-% Peressigsäure und/oder Perameisensäure, 0,1 bis 15 Gew.-% Essigsäure und/oder Ameisensäure, 20 bis 50 Gew.-% Wasserstoffperoxid, 0,1 bis 10 Gew.-% eines oder mehrerer Härtestabilisatoren, insbesondere eines solchen aus der Reihe (i), 0 bis 10 Gew.-% eines oder mehrere Zusatzstoffe aus der Reihe der Tenside, insbesondere anionischen und nichtionischen Tenside, Korrosionsinhibitoren und Puffer, 0 bis 3 Gew.-% Mineralsäure, insbesondere Schwefelsäure oder Polyphosphorsäure, 0 bis 1 Gew.-% üblichen Aktivsauerstoffstabilisatoren und Wasser in einer Menge von mindestens 40 Gew.-%.

4. Härtestabilisierende Percarbonsäurelösung nach einem der Ansprüche 1 bis 3,
**dadurch gekennzeichnet,**
**daß** sie als Percarbonsäure Peressigsäure und gegebenenfalls zusätzlich Perameisensäure in einer Gesamtmenge von 0,5 bis 6 Gew.-%, Härtestabilisatoren, insbesondere solche gemäß (i), in einer Menge von 1 bis 10 Gew.-% und 0 bis 5 Gew.-% anionische Tenside enthält.

5. Härtestabilisierende Percarbonsäurelösung nach Anspruch 3 oder 4,
**dadurch gekennzeichnet,**
**daß** sie hergestellt wurde durch Zugabe eines Härtestabilisators gemäß (i) oder einer wäßrigen Lösung desselben zu einer wäßrigen Gleichgewichtsperessigsäure mit einem Gehalt von 0,5 bis 2 Gew.-% Peressigsäure und 40 bis 50 Gew.-% Wasserstoffperoxid, wobei der Härtestabilisator herstellungsbedingt Ameisensäure in einer Menge von 1 bis 6 Gew.-%, bezogen auf das Polymer, enthält.

6. Verfahren zur Herstellung einer härtestabilisierenden Percarbonsäurelösung gemäß einem der Ansprüche 1 bis 5,
**dadurch gekennzeichnet,**
**daß** man zu einer wäßrigen Percarbonsäurelösung, insbesondere einer Gleichgewichtspercarbonsäurelösung, enthaltend Peressigsäure und/oder Perameisensäure, die zugrundeliegende/n Carbonsäure/n, Wasserstoffperoxid, Wasser, einen Mineralsäurekatalysator und übliche Aktivsauerstoffstabilisatoren, einen Härtestabilisator aus der Reihe von (i) durch oxidative Polymerisation von Acrolein oder von Acrolein und Acrylsäure hergestellten Polymeren, (ii) Polyacrylsäure, (iii) Copolymeren aus Acrylsäure und einer anderen ungesättigten Carbonsäure, insbesondere Maleinsäure, und (iv) Polymaleinsäure, wobei der Härtestabilisator unvernetzt ist, das mittlere Molekulargewicht M_{w} des Härtestabilisators im Bereich von 500 bis 25000, insbesondere 1000 bis 15000, liegt, zugibt und die Einstellung des Gleichgewichts ganz oder teilweise abwartet oder daß man einen Härtestabilisator der vorgenannten Art vor der Herstellung der Percarbonsäurelösung einem der Reaktionspartner zu deren Herstellung, also der Carbonsäure, dem Wasserstoffperoxid oder Wasser oder einem Gemisch hiervon zugibt und dann in üblicher Weise die Percarbonsäurelösung herstellt.

7. Verwendung einer härtestabilisierenden Percarbonsäurelösung gemäß einem der Ansprüche 1 bis 5 zum Vorbeugen gegen und zum Bekämpfen von schädlichen Mikroorganismen, wie Pilze, Viren, Bakterien, Hefen und Algen, sowie Würmern, Muscheln und Insekten, in wäßrigen Systemen, insbesondere Wasserkreisläufen, und zur Oberflächendesinfektion.

## Claims

1. A hardness-stabilising percarboxylic acid solution containing one or more percarboxylic acids from the group performic acid, peracetic acid and perpropionic acid, the carboxylic acid(s) from which the peracid(s) is/are derived, hydrogen peroxide, water and a hardness stabiliser from the group comprising (i) polymers prepared by the oxidative polymerisation of acrolein or of acrolein and acrylic acid, (ii) polyacrylic acid, (iii) copolymers of acrylic acid and another unsaturated carboxylic acid, in particular maleic acid, and (iv) polymaleic acid, wherein the hardness-stabiliser is not cross-linked, the average molecular weight M_{w} of the hardness stabiliser is in the range 500 to 25000, in particular 1000 to 15000, and some of the carboxyl groups in the hardness stabiliser may have been converted into percarboxyl groups.

2. A hardness-stabilising percarboxylic acid solution according to Claim 1,
**characterised in that**
it contains 0.1 to 10 wt.% of hardness stabiliser.

3. A hardness-stabilising percarboxylic acid solution according to Claim 1 or 2, consisting of: 0.1 to 15 wt.% of peracetic acid and/or performic acid, 0.1 to 15 wt.% of acetic acid and/or formic acid, 20 to 50 wt.% of hydrogen peroxide, 0.1 to 10 wt.% of one or more hardness stabilisers, in particular one from group (i), 0 to 10 wt.% of one or more additives from the group of surfactants, in particular anionic and non-ionic surfactants, corrosion inhibitors and buffers, 0 to 3 wt.% of mineral acid, in particular sulfuric acid or polyphosphoric acid, 0 to 1 wt.% of conventional active oxygen stabilisers and water in an amount of at least 40 wt.%.

4. A hardness-stabilising percarboxylic acid solution according to one of Claims 1 to 3,
**characterised in that**
it contains peracetic acid and optionally also performic acid in a total amount of 0.5 to 6 wt.% as a percarboxylic acid, hardness stabilisers, in particular those in accordance with (i), in an amount of 1 to 10 wt.% and 0 to 5 wt.% of anionic surfactants.

5. A hardness-stabilising percarboxylic acid solution according to Claim 3 or 4,
**characterised in that**
it has been prepared by adding a hardness stabiliser in accordance with (i) or an aqueous solution of the same to an aqueous equilibrium peracetic acid with a concentration of 0.5 to 2 wt.% of peracetic acid and 40 to 50 wt.% of hydrogen peroxide, wherein the hardness stabiliser, as a result of its method of preparation, contains formic acid in an amount of 1 to 6 wt.%, with respect to the polymer.

6. A process for preparing a hardness-stabilising percarboxylic acid solution according to one of Claims 1 to 5,
**characterised in that**
a hardness stabiliser from the group comprising (i) polymers prepared by the oxidative polymerisation of acrolein or of acrolein and acrylic acid, (ii) polyacrylic acid, (iii) copolymers of acrylic acid and another unsaturated carboxylic acid, in particular maleic acid, and (iv) polymaleic acid, wherein the hardness-stabiliser is not cross-linked, the average molecular weight M_{w} of the hardness stabiliser is in the range 500 to 25000, in particular 1000 to 15000 is added to an aqueous percarboxylic acid solution, in particular an equilibrium percarboxylic acid solution containing peracetic acid and/or performic acid, the carboxylic acid(s) from which this/these is/are derived, hydrogen peroxide, water, a mineral acid catalyst and conventional active oxygen stabilisers, and time is then allowed for the equilibrium to become entirely or partly established or a hardness stabiliser of the previously mentioned type is added before preparation of the percarboxylic acid solution to one of the reaction partners to be used for its preparation, that is the carboxylic acid, the hydrogen peroxide or the water or a mixture thereof and the percarboxylic acid is then prepared in a conventional manner.

7. Use of a hardness-stabilising percarboxylic acid solution in accordance with one of Claims 1 to 5 for preventing and for controlling harmful microorganisms such as moulds, viruses, bacteria, yeasts and algae, as well as worms, mussels and insects, in aqueous systems, in particular water-circulating systems, and for disinfecting surfaces.

## Revendications

1. Solution d'acide percarboxylique qui stabilise la dureté, contenant un ou plusieurs acides percarboxyliques choisis dans la série de l'acide performique, de l'acide peracétique et de l'acide perpropionique,
les acides carboxyliques sous-jacents au/aux peracides, le peroxyde d'hydrogène, l'eau et un agent stabilisant de la dureté choisi dans la série
(i) des polymères produits par polymérisation par oxydation de l'acroléine ou de l'acroléine et de l'acide acrylique,
(ii) des acides polyacryliques,
(iii) des copolymères à base d'acide acrylique et d'un autre acide carboxylique non saturé, en particulier l'acide maléique, et
(iv) l'acide polymaléique,
dans laquelle l'agent stabilisant de la dureté est non réticulé, le poids moléculaire moyen M_{w} de l'agent stabilisant de la dureté se situe dans la zone de 500 à 25 000, en particulier de 1000 à 15 000 et les groupes carboxyle de l'agent stabilisant de la dureté peuvent avoir été partiellement transformés en groupes percarboxyliques.

2. Solution d'acide percarboxylique stabilisant de la dureté selon la revendication 1,
**caractérisée en ce qu'**
elle contient de 0,1 à 10 % en poids d'agent stabilisant de la dureté.

3. Solution d'acide percarboxylique stabilisant la dureté selon la revendication 1 ou la revendication 2,
qui consiste en
0,1 à 15 % en poids d'acide peracétique et/ou d'acide performique,
0,1 % à 15 % d'acide acétique et/ou d'acide formique,
20 % à 50 % en poids de peroxyde d'hydrogène,
0,1 à 10 % en poids d'un ou plusieurs agents stabilisants de la dureté, en particulier un tel choisi dans la série (i),
0 à 10 % en poids d'un ou plusieurs additifs choisis dans la série des agents tensioactifs, en particulier des agents tensioactifs anioniques et non ioniques, des inhibiteurs de corrosion et des tampons,
0 à 3 % en poids d'acide minéral, en particulier l'acide sulfurique ou l'acide polyphosphorique,
0 à 1 % d'agents stabilisants de l'oxygène actif et de l'eau, en une quantité d'au moins 40 % en poids.

4. Solution d'acide percarboxylique stabilisant de la dureté selon l'une des revendications 1 à 3,
**caractérisée en ce qu'**
elle contient comme acide percarboxylique, de l'acide peracétique et, le cas échéant, en supplément de l'acide performique en une quantité totale de 0,5 à 6 % en poids, des agents stabilisateurs de la dureté, en particulier ceux conformément à (i) en une quantité de 1 à 10 % en poids, et de 0 à 5 % en poids d'agents tensioactifs anioniques.

5. Solution d'acide percarboxylique stabilisant de la dureté selon la revendication 3 ou la revendication 4,
**caractérisée en ce qu'**
elle a été préparée par addition d'un agent stabilisant conformément à (i) ou d'une solution aqueuse de celui-ci à un acide peracétique d'équilibre, aqueux, ayant une teneur de 0,5 à 2 % en poids d'acide peracétique et de 40 à 50 % en poids de peroxyde d'hydrogène dans lequel l'agent stabilisant de la dureté contient en fonction de la préparation de l'acide formique en une quantité de 1 à 6 % en poids - rapporté au polymère.

6. Procédé de préparation d'une solution d'acide percarboxylique stabilisant de la dureté conformément à l'une des revendications 1 à 5,
**caractérisé en ce qu'**
on ajoute à une solution d'acide percarboxylique aqueuse, en particulier à une solution d'acide percarboxylique d'équilibre contenant de l'acide peracétique et/ou de l'acide performique, les acide(s) carbolyxiques sous-jacent(s), du peroxyde d'hydrogène, de l'eau, un catalyseur à base d'acide minéral et des agents stabilisants et d'oxygène actif, un agent stabilisant de la dureté choisi dans la série de (i), des polymères produits par polymérisation par oxydation de l'acroléine ou de l'acroléine et de l'acide acrylique, (ii) de l'acide polyacrylique, (iii) des copolymères à base d'acide acrylique et d'un autre acide carboxylique non saturé, en particulier de l'acide maléique et (iv) de l'acide polymaléique, pour lesquels l'agent stabilisant de la dureté est non réticulé, le poids moléculaire moyen Mw de l'agent stabilisateur de la dureté se situe dans la zone de 500 à 25 000, en particulier de 1000 à 15 000 et on attend l'ajustement de l'équilibre totalement ou partiellement, ou on ajoute un agent stabilisant de la dureté du type précité avant la préparation de la solution d'acide percarboxylique à l'un des partenaires de la réaction pour leur préparation, par conséquence à l'acide carboxylique, au peroxyde d'hydrogène ou à l'eau, ou à un mélange de ceux-ci et ensuite on prépare d'une manière usuelle, la solution d'acide percarboxylique.

7. Utilisation d'une solution d'acide percarboxylique qui stabilise la dureté, conformément à l'une des revendications 1 à 5 en vue de prévenir et de détruire les microorganismes nocifs comme des champignons, des virus, des bactéries, des levures et des algues, ainsi que des vers, des coquillages et des insectes, dans des systèmes aqueux, en particulier des circuits d'eau, et en vue de la désinfection des surfaces.
